# EUROPEAN PATENT APPLICATION

(11) **EP 1 004 412 A1**
(43) Date of publication of application: **31.05.2000**
(21) Application number: 98122263.1
(22) Date of filing: 24.11.1998
(51) Int. Cl.: B26F 1/26, B29C 59/04, A61F 13/15

(54) **Process and apparatus for making selectively apertured web materials**

(71) Applicant: THE PROCTER & GAMBLE COMPANY, Cincinnati, Ohio 45202 (US)
(72) Inventor: Fuchs, Chistofer, 61476 Kronberg (DE); Pescher, Georg, 65812 Bad Soden (DE)
(74) Representative: Kohol, Sonia

(57) **Abstract**

The present invention provides a method for selectively aperturing a web material. The web material is weakened along a plurality of regions and then at least a portion of the web material comprised in the weakened regions is removed. Thus, a plurality of apertures in the web material is obtained.

## Description

### FIELD OF THE INVENTION

The present invention relates to apertured web materials, a method of making the same, and an apparatus for making the same. Apertured web materials are particularly well suited for use in disposable absorbent articles such as diapers, incontinence briefs, training pants, feminine hygiene garments, and the like.

### BACKGROUND

Web materials such as for example nonwoven webs, films, and the like may be manufactured into products and components of products so inexpensively that the products could be viewed as disposable after only one or a few uses. Representatives of such products include disposable absorbent articles, such as diapers, incontinence briefs, training pants, feminine hygiene garments, and the like.

Infants and other incontinent individuals wear disposable absorbent articles such as diapers to receive and contain urine and other body exudates. Absorbent articles function both to contain the discharged materials and to isolate these materials from the body of the wearer and from the wearer's garments and bed clothing. Disposable absorbent articles having many different basic designs are known to the art.

A typical absorbent article includes a liquid pervious topsheet, a liquid impervious backsheet joined to the topsheet, and an absorbent core positioned between the topsheet and the backsheet. web materials are often used as the topsheet because they are liquid pervious and provide a skin friendly surface. However, in certain uses web materials do not function all that well as a topsheet as body exudates sometimes hang-up or get caught in the web material and thus, become trapped against the wearers skin. One solution to the aforementioned problem is to provide apertures in the web material so that body exudates may readily penetrate through the web material and into the underlying absorbent core.

Unfortunately, certain techniques used to form apertured web materials are either costly, create an undesirable harsh feeling against the wearers skin, or are subject to tearing, particularly when the apertured web material is to be used as a topsheet on a disposable absorbent article.

To overcome the problems of the prior art web material, a process for making selectively apertured nonwoven webs has been taught in PCT publication No. WO97/11662. In this process, a nonwoven web is weakened at a plurality of regions and then a tensioning force is applied to the nonwoven web causing the nonwoven to rupture at the plurality of weakened regions creating a plurality of apertures in the nonwoven web coincident with the weakened regions. Hence, during this process, no material is removed from the nonwoven web.

The present invention provides an alternative process for making an apertured web material by removing web material from the weakened regions. The present invention further provides an apparatus for making the apertured web material, and a disposable absorbent article comprising the same.

### SUMMARY OF THE INVENTION

The present invention provides a process for selectively aperturing a web material. The process comprises the steps of providing a web material, of selectively weakening a plurality of regions of said web material, and of removing at least a portion of the web material comprised in said weakened regions using a technique selected from the group of gas blowing and pulling.

It is another aspect of the present invention to provide an apparatus for selectively aperturing a web material. The apparatus comprises a web material supply means, a web weakening means for selectively weakening a plurality of regions of said web material, and a web removal means for removing at least a part of said web material comprised in said weakened regions.

It is another object of the present invention to provide an absorbent article comprising the selective apertured web material manufactured according to the process of the present invention.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which like designations are used to designate substantially identical elements, and in which:
FIG. 1 is a schematic representation of an exemplary process for selectively aperturing a nonwoven web of the present invention;
FIG. 2 is an enlarged perspective illustration of a web weakening means of the apparatus of the present invention;
FIG. 3 is an enlarged perspective illustration of a web removal system of an apparatus of the present invention;
FIG. 4 is a plan view of a disposable diaper embodiment of the present invention having portions cut away to reveal underlying structure, the inner surface of the diaper is facing the viewer.

### DETAILED DESCRIPTION OF THE INVENTION

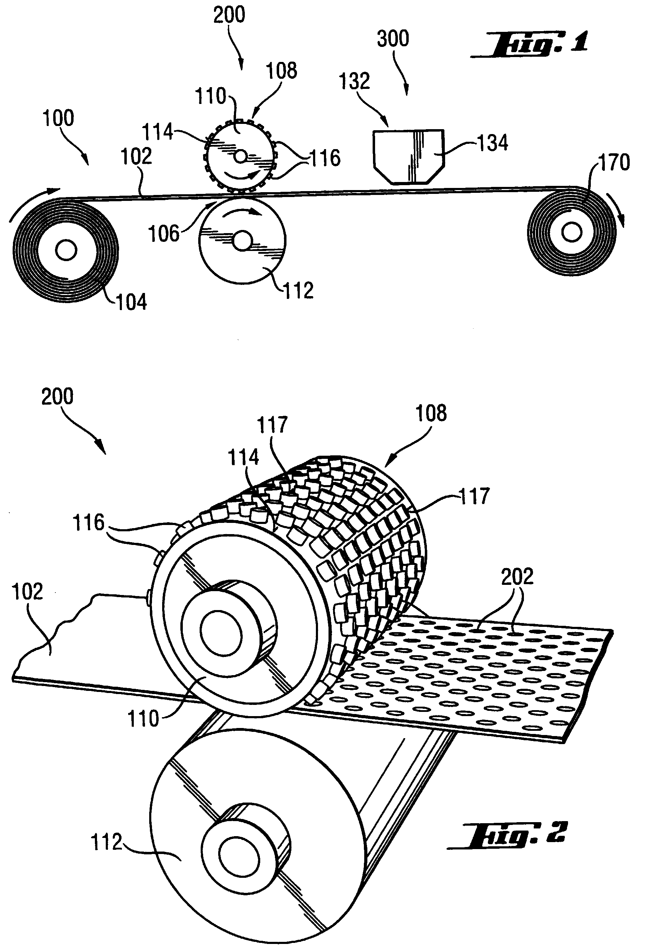

Referring to FIG. 1 there is schematically illustrated at 100 a process for selectively aperturing a web material suitable, for example, for use as a topsheet on a disposable absorbent article.

As used herein, the term " web material" refers to a sheet-like material made from thermoplastic material, or to a composite or laminate comprising two or more such sheet-like materials. For example, a web material can be a fibrous web, a non-fibrous web, or the like. A web material suitable for the present invention is essentially two-dimensional, i.e. the thickness of the web material is much smaller than its longitudinal and its transverse dimension. Additionally, the transverse dimension of the web material is substantially smaller than its longitudinal dimension. The longitudinal dimension preferably exceeds the transverse dimension by a factor of 100, most preferably the longitudinal dimension of the web material of the present invention is essentially infinite.

As used herein, the term "fibrous web" refers to a web that has a structure of individual thermoplastic fibers or threads which are interlaid such as a nonwoven web, a woven web, a knit web, or the like. The fibrous webs of the present invention may be absorbent or non-absorbent, liquid pervious, or liquid impervious.

As used herein, the term " non-fibrous webs" refers to a web that has a uniform structure such as a film and is made from a thermoplastic material.

According to the process of the present invention, a non-apertured web material is supplied to the process. In one embodiment of the present invention, the non-apertured web material 102 is unwound from a supply roll 104 and travels in a direction indicated by the arrows associated therewith as the supply roll 104 rotates in the direction indicated by the arrows associated therewith. Alternatively, the web material 102 may be formed on-line, for example, by known nonwoven extrusion processes, such as, for example, known meltblowing processes or known spunbonding processes, and passed directly through the nip 106 without first being bonded and/or stored on a supply roll.

In one embodiment of the present invention, the web material is a nonwoven web. As used herein, the term "nonwoven web", refers to a web that has a structure of individual thermoplastic fibers or threads which are interlaid, but not in any regular, repeating manner. The nonwoven web 102 may be extensible, elastic, or non-elastic. The nonwoven web 102 may be a spunbonded web, a meltblown web, or a bonded carded web. If the nonwoven web is a web of meltblown fibers, it may include meltblown microfibers. The nonwoven web 102 may be made of fiber forming polymers such as, for example, polyolefins. Exemplary polyolefins include one or more of polypropylene, polyethylene, ethylene copolymers, propylene copolymers, butene copolymers, polyamides, bicomponent, polyethylene-terephthalate, combinations thereof, and the like. In another embodiment, the nonwoven can be a multilayer web having, for example, a first carded web having a basis weight between 5 grams per square meter and 100grams per square meter, preferably between 5 grams per square meter and 60 grams per square meter, and a second carded web having a basis weight between 5 grams per square meter and 100 grams per square meter, preferably between 5 grams per square meter and 60 grams per square meter. In another embodiment, the nonwoven web 102 may be a multilayer material having, for example, at least one layer of a spunbonded web joined to at least one layer of a meltblown web, a bonded carded web, or other suitable material. For example, the nonwoven web 102 may be a multilayer web having a first layer of spunbonded polypropylene having a basis weight from about 5 to about 100 grams per square meter, a layer of meltblown polypropylene having a basis weight from about 5 to about 100 grams per square meter, and a second layer of spunbonded polypropylene having a basis weight from about 5 to about 200 grams per square meter. Alternatively, the nonwoven web may be a single layer of material, such as, for example, a spunbonded web having a basis weight from about 5 to about 300 grams per square meter or a meltblown web having a basis weight from about 5 to about 200 grams per square meter. The nonwoven web may be joined to a polymeric film to form a laminate. The nonwoven web 102 may also be a composite made up of a mixture of two or more different fibers or a mixture of fibers and particles. Such mixtures may be formed by adding fibers and/or particulates to the gas stream in which the meltblown fibers or spunbond fibers are carried so that an intimate entangled co-mingling of fibers and other materials, e.g., wood pulp, staple fibers and particles occurs prior to collection of the fibers. The nonwoven web of fibers should be joined by bonding to form a coherent web structure. Suitable bonding techniques include, but are not limited to air-through bonding, chemical bonding, thermobonding, such as point calendering, hydroentangling, and needling.

As used herein, the term "microfibers", refers to small diameter fibers having an average diameter not greater than about 100 microns.

As used herein, the term "meltblown fibers", refers to fibers formed by extruding a molten thermoplastic material, through a plurality of fine, usually circular, die capillaries as molten threads or filaments into a high velocity gas (e.g., gas) stream which attenuates the filaments of molten thermoplastic material to reduce their diameter, which may be to a microfiber diameter. Thereafter, the meltblown fibers are carded by the high velocity gas stream and are deposited on a collecting surface to form a web of randomly dispersed meltblown fibers.

As used herein, the term "spunbonded fibers", refers to small diameter fibers which are formed by extruding a molten thermoplastic material as filaments from a plurality of fine, usually circular, capillaries of a spinneret with the diameter of the extruded filaments then being rapidly reduced as by, for example, eductive drawing or other well-known spunbonding mechanisms.

As used herein, the term "polymer" generally includes, but is not limited to, hompolymers, copolymers, such as, for example, block, graft, random and alternating copolymers, terpolymers, etc., and blends and modifications thereof. Furthermore, unless otherwise specifically limited, the term "polymer" shall include all possible geometrical configurations of the material. These configurations include, but are not limited to, isotactic, syndiotactic and random symmetries.

In another embodiment of the present invention, the web material is a polymeric film material. Suitable polymeric film materials include but are not limited to polyolefins, such as polyethylenes, polypropylene, ethylene copolymers, propylene copolymers, and butene copolymers; nylon (polyamide); metallocene catalyst-based polymers; poly (methyl methacrylate); polystyrene; poly (vinyl chloride); polyester; polyurethane; compatible polymers; compatible copolymers; and blends, laminates and/or combinations thereof.

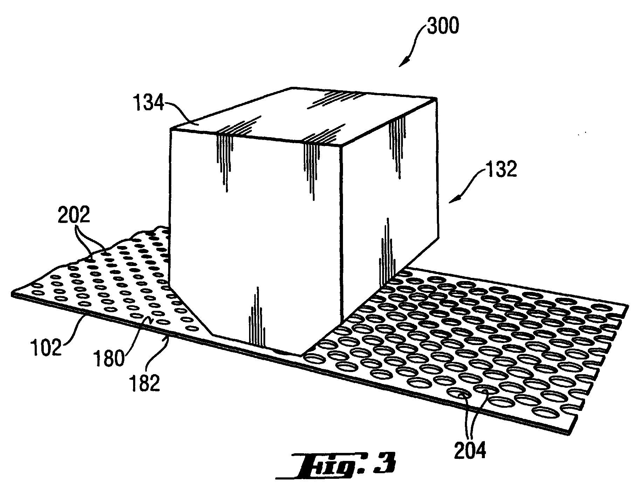

Subsequent to being supplied to the process, a plurality of regions 202 of the web material 102 is selectively weakened 200 as is shown in FIG. 2.

In one embodiment of the process of the present invention, the step of selectively weakening a plurality of regions 202 of the web material 102 comprises a step of selectively heating the plurality of regions 202. The regions 202 can be heated by applying electromagnetic waves including but not limited to infrared and LASER, pressure, ultrasonic waves, or a combination thereof. In one embodiment of the present invention, the regions 202 are only preheated to a temperature below the melting point of the web material. In this case, the generation of potentially sharp edges of molten material is reduced. Alternatively, the step of selectively weakening the regions 202 of the web material 102 may comprise a step of selectively applying a chemical agent to the regions 202. For example, the strength of the web material in the regions 202 may be reduced via that agent.

In one embodiment of the process of the present invention, the weakened regions of the web material may circumscribe substantially less weakened or even unweakened regions. For example, the weakened regions may take the form of a hollow circle, ellipse, square, or the like.

In one embodiment of the process of the present invention, the web material outside of the weakened regions may be melt-stabilized by applying a pressure simultaneously with the step of weakening.

Subsequent to the step 200 of weakening a plurality of regions of the web material, at least a portion of the web material comprised in the weakened web regions 202 is removed 300 using a technique selected from the group of gas blowing, pulling. After removal of the web material, a plurality of apertures 204 remains in the web material 102 at the locations of the weakened regions 202. The size of the apertures 204 may differ somewhat from the size of the weakened regions depending on the process settings and the web material properties. In case the weakened regions circumscribe substantially less weakened regions, at least a portion of the web material comprised in the less weakened regions may be removed along with the weakened web material.

In one embodiments of the apparatus of the present invention, a uniform pressure is applied to the upper major surface 180 of the web material by blowing gas through the web from the upper major surface 180 to the lower major surface 182. Since the web material comprises a plurality of weakened regions 202 upon arrival on the web removal means, these regions 202 are more prone to failure under the applied pressure than the untreated regions. The gas flux through web material 102 (and thus the pressure exerted onto the surface 180 of the web material) has to be chosen such that at least a portion of the web material comprised in the weakened regions 202 is removed from the web material and is carried away with the gas. Simultaneously, the gas flux must also be chosen such that at least a part of the web integrity is maintained after the removal of the web material from the weakened regions 202. In one embodiment of the process of the present invention, the gas flow may be directed selectively through the weakened regions 202 of the web material 102.

In an alternative embodiment of the present invention, at least a part of the web material comprised in the weakened regions 202 may be removed by pulling, i.e. by the exertion of a physical force onto the web material to be removed. In this case, the pull force has to be chosen such that at least a portion of the web material comprised in the weakened regions 202 is removed from the web and such that at least a part of the web integrity is maintained after the removal of the web material from the weakened regions 202.

The uni-directionality of the removal process of the web material from the weakened regions may impose a uni-directionality of the resulting aperture edges. For example, some web material such as fibers that has been in close proximity to weakened web material before the removal of the latter may be oriented towards the lower major surface by the removal process. This web material may even project from the lower major surface, thereby defining the edge of a three-dimensional aperture.

After the web material has been removed from the weakened regions, the web material 102 may be taken up on wind-up roll 180 and stored. Alternatively, the web material 102 may be fed directly to a production line where it is used to form a topsheet on a disposable absorbent article.

It is another aspect of the present invention to provide an apparatus for making a selectively apertured web material according to the present invention. The apparatus of the present invention comprises a web material supply means, a web weakening means 108 for selectively weakening a plurality of regions of said web material, and a web removal means 132 for removing at least a part of said web material comprised in said weakened regions.

In one embodiment of the apparatus of the present invention, the web material supply means comprises a supply roll 104 from which the web material 102 is unwound. Alternatively, the web material supply means may comprise a web material production means such as a nonwoven extrusion system which produces the web material online.

In one embodiment of the apparatus of the present invention, the web weakening means is formed by rollers 110 and 112. In this case the web material 102 is passed through the nip 106. Referring now to the embodiment of the present invention shown in FIGS. 1 and 2, the web weakening means 108 may comprise a patterned calander roller 110 and a smooth anvil roller 112. One or both of the patterned calander roller 110 and the smooth anvil roller 112 may be heated and the pressure between the two rollers may be adjusted by well known means to provide the desired temperature, if any, and pressure to concurrently weaken and melt-stabilize the web material 102 at a plurality of locations.

The patterned calendar roller 110 is configured to have a circular cylindrical surface 114, and a plurality of protuberances or pattern elements 116 which extend outwardly from surface 114. The protuberances 116 are disposed in a predetermined pattern with each protuberance 116 being configured and disposed to precipitate a weakened, melt-stabilized location in the web material 102 to effect a predetermined pattern of weakened, melt-stabilized locations in the web material 102.

As shown in FIG. 2, patterned calander roller 110 has a repeating pattern of protuberances 116 which extend about the entire circumference of surface 114. Alternatively, the protuberances 116 may extend around a portion, or portions of the circumference of surface 114. The protuberances 116 are preferably truncated conical shapes which extend radially outwardly from surface 114 and which have circular distal end surfaces 117. Although it is not intended to thereby limit the scope of the present invention to protuberances of only this configuration. Other suitable shapes for distal ends 117 include, but are not limited to elliptical, square, rectangular, etc. The protuberances may also be designed such that at least a portion of the protuberances comprise at least one aperture on their distal ends. In this case, at least a part of the weakened regions of the web material circumscribes a substantially less preheated region. Such a design may support the subsequent removal of the web material from the weakened regions since the web material to be removed then comprises a non-weakened part which potentially enhances the overall integrity of the material to be removed.

The roller 110 is finished so that all of the end surfaces 117 lie in an imaginary right circular cylinder which is coaxial with respect to the axis of rotation of roller 110.

The protuberances 116 are disposed in a regular predetermined pattern of rows and columns in the embodiment shown in FIG. 2, although it is not intended to thereby limit the scope of the present invention to the pattern of protuberances of only this configuration. The protuberances may be disposed in any predetermined pattern about patterned calander roll 110.

In one embodiment of the present invention the anvil roller 112, is a smooth surfaced, circular cylinder of steel.

From the web weakening means 108, the web material 102 passes through a web material removal system 132. The web material removal means removes at least a portion of the web material comprised in the weakened regions 202 of the web material. In case the weakened regions circumscribe substantially less weakened regions, at least a portion of these non-weakened regions may be removed along with the web material from the weakened regions.

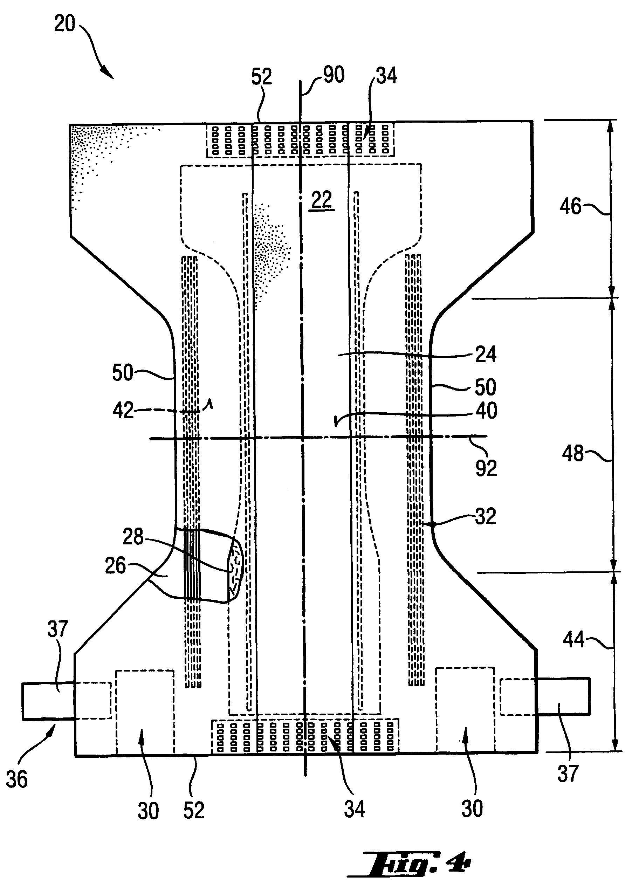

Referring to FIG. 3 now, the web material removal system may be a an gas knife 134 blowing gas from the upper major surface 180 of the web material to the lower major surface 182. Thus, at least a portion of the web material comprised in the weakened regions 202 is removed when the web material is exerted to the gas flow. The gas blown through the web material may be heated in order to support the removal of web material. The web material removal system may further comprise a receptacle for the removed web material preferably capable of filtering the web material from the gas flow.

Alternatively, the web material removal system may comprise a sticky calander roll and a smooth anvil roll. The surface of the calander roll is treated such that at least a portion of the web material comprised in the weakened regions adheres to the surface of the calander roll. Through the rotation of the calander roll during the process, at least a portion of the web material comprised in the weakened regions is removed from the web material. The removed web material can be removed from the surface of the calander roll by a doctor blade or the like.

In another alternative embodiment of the present invention, the anvil roll 112 of the web weakening means 108 has a sticky surface. Thus, at least a portion of the web material comprised in the weakened regions 202 adheres to the anvil roll 112 and is thus removed from the web material immediately after selective weakening of the web material.

In another alternative embodiment of the apparatus of the present invention, the web removal means may be integrated into the web weakening means. In one embodiment, an air flow through apertured protuberances of the calander roll may be generated such that the weakened web material is weakened simultaneously with or immediately after weakening at least a portion of the web material comprised in the weakened regions is removed from the web material. The air flow through the protuberances may be directed towards the web material or it may be away from the web material. Alternatively, the anvil roll of the web weakening means may comprise apertures to direct air flow from or to the web material in order to remove at least a part of the web material comprised in the weakened regions. Apertures in the protuberances of the calander roll and in the respective anvil roll may also be used to generate an air flow selectively through at least a part the weakened regions.

It is another aspect of the present invention to provide a disposable absorbent article comprising a selectively apertured web material according to the present invention.

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and liner. A preferred embodiment of an absorbent article of the present invention is the unitary disposable absorbent article, diaper 20, shown in FIG. 4. As used herein, the term "diaper" refers to an absorbent article generally worn by infants and incontinent persons that is worn about the lower torso of the wearer. It should be understood, however, that the present invention is also applicable to other absorbent articles such as incontinence briefs, incontinence undergarments, diaper holders and liners, feminine hygiene garments, training pants, and the like.

FIG. 4 is a plan view of the diaper 20 of the present invention in its flat-out, uncontracted state (i.e., with elastic induced contraction pulled out) with portions of the structure being cut-away to more clearly show the construction of the diaper 20 and with the portion of the diaper 20 which faces the wearer, the inner surface 40, facing the viewer. As shown in FIG. 4, the diaper 20 preferably comprises a containment assembly 22 comprising a liquid pervious topsheet 24; a liquid impervious backsheet 26 joined to the topsheet; and an absorbent core 28 positioned between the topsheet 24 and the backsheet 26. The diaper preferably further comprises elasticized side panels 30; elasticized leg cuffs 32; elasticized waistbands 34; and a fastening system 36 preferably comprising a pair of securement members 37 and a landing member, (not shown).

The diaper 20 is shown in FIG. 4 to have an inner surface 40 (facing the viewer in FIG. 4), an outer surface 42 opposed to the inner surface 40, a rear waist region 44, a front waist region 46 opposed to the rear waist region 44, a crotch region 48 positioned between the rear waist region 44 and the front waist region 46, and a periphery which is defined by the outer perimeter or edges of the diaper 20 in which the longitudinal edges are designated 50 and the end edges are designated 52. The inner surface 40 of the diaper 20 comprises that portion of the diaper 20 which is positioned adjacent to the wearer's body during use (i.e., the inner surface 40 generally is formed by at least a portion of the topsheet 24 and other components joined to the topsheet 24). The outer surface 42 comprises that portion of the diaper 20 which is positioned away from the wearer's body (i.e., the outer surface 42 is generally formed by at least a portion of the backsheet 26 and other components joined to the backsheet 26). As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. The rear waist region 44 and the front waist region 46 extend from the end edges 52 of the periphery to the crotch region 48.

The diaper 20 also has two centerlines, a longitudinal centerline 90 and a transverse centerline 92. The term "longitudinal", as used herein, refers to a line, axis, or direction in the plane of the diaper 20 that is generally aligned with (e.g. approximately parallel with) a vertical plane which bisects a standing wearer into left and right halves when the diaper 20 is worn. The terms "transverse" and "lateral", as used herein, are interchangeable and refer to a line, axis or direction which lies within the plane of the diaper that is generally perpendicular to the longitudinal direction (which divides the wearer into front and back body halves).

The containment assembly 22 of the diaper 20 is shown in FIG. 4 as comprising the main body (chassis) of the diaper 20. The containment assembly 22 comprises at least topsheet 24, backsheet 26 and absorbent core 28. When the absorbent article comprises a separate holder and a liner, the containment assembly 22 generally comprises the holder and the liner (i.e., the containment assembly 22 comprises one or more layers of material to define the holder while the liner comprises an absorbent composite such as a topsheet, a backsheet, and an absorbent core.) For unitary absorbent articles, the containment assembly 22 comprises the main structure of the diaper with other features added to form the composite diaper structure. Thus, the containment assembly 22 for the diaper 20 generally comprises the topsheet 24, the backsheet 26, and the absorbent core 28.

FiG. 4 shows a preferred embodiment of the containment assembly 22 in which the topsheet 24 and the backsheet 26 have length and width dimensions generally larger than those of the absorbent core 28. The topsheet 24 and the backsheet 26 extend beyond the edges of the absorbent core 28 to thereby form the periphery of the diaper 20. While the topsheet 24, the backsheet 26, and the absorbent core 28 may be assembled in a variety of well known configurations, exemplary containment assembly configurations are described generally in U.S. Pat. No. 3,860,003 entitled "Contractible Side Portions for Disposable Diaper" which issued to Kenneth B. Buell on Jan. 14, 1975; and U.S. Pat. No. 5,151,092 entitled "Absorbent Article With Dynamic Elastic Waist Feature Having A Predisposed Resilient Flexural Hinge" which issued to Kenneth B. Buell et al., on Sep. 29, 1992; each of which is incorporated herein by reference. The absorbent core 28 may be any absorbent member which is generally compressible, conformable, non-irritating to the wearer's skin, and capable of absorbing and retaining liquids such as urine and other certain body exudates. As shown in FIG. 4, the absorbent core 28 has a garment-facing side, a body-facing side, a pair of side edges, and a pair of waist edges. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 28 may vary (e.g., the absorbent core may have varying caliper zones, a hydrophilic gradient, a superabsorbent gradient, or lower average density and lower average basis weight acquisition zones; or may comprise one or more layers or structures). Further, the size and absorbent capacity of the absorbent core 28 may also be varied to accommodate wearers ranging from infants through adults. However, the total absorbent capacity of the absorbent core 28 should be compatible with the design loading and the intended use of the diaper 20.

One embodiment of the diaper 20 has an asymmetric, modified T-shaped absorbent core 28 having ears in the front waist region but a generally rectangular shape in the rear waist region. Exemplary absorbent structures for use as the absorbent core 28 of the present invention that have achieved wide acceptance and commercial success are described in U.S. Pat. No. 4,610,678 entitled "High-Density Absorbent Structures" issued to Weisman et al. on Sep. 9, 1986; U.S. Pat. No. 4,673,402 entitled "Absorbent Articles With Dual-Layered Cores" issued to Weisman et al. on Jun. 16, 1987; U.S. Pat. No. 4,888,231 entitled "Absorbent Core Having A Dusting Layer' issued to Angstadt on Dec. 19, 1989; and U.S. Pat. No. 4,834,735, entitled "High Density Absorbent Members Having Lower Density and Lower Basis Weight Acquisition Zones", issued to Alemany et al. on May 30, 1989. The absorbent core may further comprise the dual core system containing an acquisition/distribution core of chemically stiffened fibers positioned over an absorbent storage core as detailed in U.S. Pat. No. 5,234,423, entitled "Absorbent Article With Elastic Waist Feature and Enhanced Absorbency" issued to Alemany et al., on Aug. 10, 1993; and in U.S. Pat. No. 5,147,345, entitled "High Efficiency Absorbent Articles For Incontinence Management" issued to Young, LaVon and Taylor on Sep. 15, 1992. All of these patents are incorporated herein by reference.

The backsheet 26 is positioned adjacent the garment-facing surface of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1258. An example of a suitable attachment means comprising an open pattern network of filaments of adhesive is disclosed in U.S. Pat. No. 4,573,986 entitled "Disposable Waste-Containment Garment", which issued to Minetola et al. on Mar. 4, 1986. Another suitable attachment means comprising several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art. Embodiments of the present invention are also contemplated wherein the absorbent core is not joined to the backsheet 26, the topsheet 24, or both in order to provide greater extensibility in the front waist region 46 and the rear waist region 44.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as bedsheets and undergarments, however, the backsheet 26 may permit vapors to escape from the absorbent core 28 (i.e., is breathable). Thus, the backsheet 26 preferably comprises a polymeric film such as thermoplastic films of polyethylene or polypropylene. A suitable material for the backsheet 26 is a thermoplastic film having a thickness of from about 0.012 mm (0.5 mil) to about 0.051 mm (2.0 mils). The topsheet 24 is positioned adjacent the body-facing surface of the absorbent core 28 and is preferably joined thereto and to the backsheet 26 by attachment means (not shown) such as those well known in the art. Suitable attachment means are described with respect to joining the backsheet 26 to the absorbent core 28. In a preferred embodiment of the present invention, the topsheet 24 and the backsheet 26 are joined directly to each other in the diaper periphery and are indirectly joined together by directly joining them to the absorbent core 28 by the attachment means (not shown).

The topsheet 24 is preferably compliant, soft feeling, and non-irritating to the wearer's skin. Further, the topsheet 24 is preferably liquid pervious permitting liquids (e.g., urine) to readily penetrate through its thickness. A suitable topsheet 24 may be manufactured from the apertured web material according to the present invention.

The diaper 20 preferably further comprises elasticized leg cuffs 32 for providing improved containment of liquids and other body exudates. Each elasticized leg cuff 32 may comprise several different embodiments for reducing the leakage of body exudates in the leg regions. (The leg cuff can be and is sometimes also referred to as leg bands, side flaps, barrier cuffs, or elastic cuffs.) U.S. Pat. No. 3,860,003 describes a disposable diaper which provides a contractible leg opening having a side flap and one or more elastic members to provide an elasticized leg cuff (gasketing cuff). U.S. Pat. No. 4,909,803 entitled "Disposable Absorbent Article Having Elasticized Flaps" issued to Aziz et al. on Mar. 20, 1990, describes a disposable diaper having "stand-up" elasticized flaps (barrier cuffs) to improve the containment of the leg regions. U.S. Pat. No. 4,695,278 entitled "Absorbent Article Having Dual Cuffs" issued to Lawson on Sep. 22, 1987, describes a disposable diaper having dual cuffs including a gasketing cuff and a barrier cuff. U.S. Pat. No. 4,704,115 entitled "Disposable Waist Containment Garment" issued to Buell on Nov. 3, 1987, discloses a disposable diaper or incontinence garment having side-edge-leakage-guard gutters configured to contain free liquids within the garment. Each of these patents are incorporated herein by reference. While each elasticized leg cuff 32 may be configured so as to be similar to any of the leg bands, side flaps, barrier cuffs, or elastic cuffs described above, it is preferred that each elasticized leg cuff 32 comprise at least an inner barrier cuff comprising a barrier flap and a spacing element such as described in the above-referenced U.S. Pat. No. 4,909,803. In a preferred embodiment, the elasticized leg cuff 32 additionally comprises an elastic gasketing cuff with one or more elastic strands, positioned outboard of the barrier cuff such as described in the above-referred U.S. Pat. No. 4,695,278.

The diaper 20 preferably further comprises an elasticized waistband 34 that provides improved fit and containment. The elasticized waistband 34 is that portion or zone of the diaper 20 which is intended to elastically expand and contract to dynamically fit the wearer's waist. The elasticized waistband 34 preferably extends longitudinally outwardly from at least one of the waist edges of the absorbent core 28 and generally forms at least a portion of the end edge of the diaper 20. Disposable diapers are generally constructed so as to have two elasticized waistbands, one positioned in the rear waist region and one positioned in the front waist region, although diapers can be constructed with a single elasticized waistband. Further, while the elasticized waistband 34 or any of its constituent elements can comprise a separate element affixed to the diaper 20, the elasticized waistband 34 may be constructed as an extension of other elements of the diaper such as the backsheet 26 or the topsheet 24, preferably both the backsheet 26 and the topsheet 24. The elasticized waistband 34 may be constructed in a number of different configurations including those described in U.S. Pat. No. 4,515,595 entitled "Disposable Diapers with Elastically Contractible Waistbands" issued to Kievit et al. on May 7, 1985 and the above referenced U.S. Pat. No. 5,151,092 issued to Buell; each of these references being incorporated herein by reference.

In a preferred embodiment, the diaper 20 also comprises elasticized side panels 30 disposed in the rear waist region 44. (As used herein, the term "disposed" is used to mean that an element(s) of the diaper is formed (joined and positioned) in a particular place or position as a unitary structure with other elements of the diaper or as a separate element joined to another element of the diaper.) The elasticized side panels 30 provide an elastically extensible feature that provides a more comfortable and contouring fit by initially conformably fitting the diaper to the wearer and sustaining this fit throughout the time of wear well passed when the diaper has been loaded with exudates since the elasticized side panels allow the sides of the diaper to expand and contract. The elasticized side panels 30 further provide more effective application of the diaper 20 since even if the diaperer pulls one elasticized side panel 30 farther than the other during the application (asymmetrically), the diaper 20 will "self-adjust" during wear. While the diaper 20 of the present invention preferably has the elasticized side panels 30 disposed in the rear waist region 44; alternatively, the diaper 20 may also be provided with elasticized side panels disposed in the front waist region 46 and the rear waist region 44. While the elasticized side panels 30 may be constructed in a number of configurations, examples of diapers with elasticized side panels are disclosed in U.S. Pat. No. 4,857,067, entitled "Disposable Diaper Having Shirred Ears" issued to Wood, et al. on Aug. 15, 1989; U.S. Pat. No. 4,381,781 issued to Sciaraffa, et al. on May 3, 1983; U.S. Pat. No. 4,938,753 issued to Van Gompel, et al. on Jul. 3, 1990; and in U.S. Pat. No. 5,151,092 issued to Buell et al. on Sep. 29, 1992; each of which are incorporated herein by reference. Suitable elastic materials for use as the elasticized side panels include elastomeric foams, synthetic or natural rubber, synthetic or natural rubber foams, elastomeric films, elastomeric scrim, elastomeric woven or nonwoven webs, elastomeric composites such as elastomeric nonwoven laminates, or the like.

The diaper 20 also comprises a fastening system 36 which forms a side closure which maintains the rear waist region 44 and the front waist region 46 in an overlapping configuration such that lateral tensions are maintained around the circumference of the diaper to maintain the diaper on the wearer. Exemplary fastening systems are disclosed in U.S. Pat. No. 4,869,724 issued to Scripps on Sep. 26, 1989; U.S. Pat. No. 4,846,815 issued to Scripps on Jul. 11, 1989; U.S. Pat. No. 4,894,060 issued to Nestegard on Jan. 16, 1990; U.S. Pat. No. 4,946,527 issued to Battrell on Aug. 7, 1990; U.S. Pat. No. 3,848,594 issued to Buell on Nov. 19, 1974; and U.S. Pat. No. 4,662,875 issued to Hirotsu and Robertson on May 5, 1987. Each of these patents are incorporated herein by reference.

The selectively apertured web material of the present invention may also be used as a topsheet on other disposable absorbent articles such as for example, incontinence briefs, training pants, feminine hygiene garments and the like.

The selectively apertured web material may also be used in other portions of a disposable absorbent article such as, for example, as an acquisition layer positioned between the topsheet and the absorbent core, as part of the absorbent core, or as portions of other components of the disposable absorbent article.

## Claims

1. A process for selectively aperturing a web material, said process comprising the steps of:
- providing a web material
- selectively weakening a plurality of regions of said web material
- removing at least a portion of the web material comprised in said weakened regions using a technique selected from the group of: gas blowing, pulling.

2. A process for selectively aperturing a web material according to Claim 1, wherein said step of selectively weakening a plurality of regions of said web material comprises a step of selectively heating said plurality of regions.

3. A process for selectively aperturing a web material according to Claim 2 wherein said regions are heated to a temperature below the melting point of said web material.

4. A process for selectively aperturing a web material according to Claim 1, said web having an upper major surface and a lower major surface, wherein said step of removing web material comprises blowing gas through said web material from said upper major surface to said lower major surface in order to aperture at least a portion of said weakened regions.

5. A process for selectively aperturing a web material according to Claim 1 wherein at least a part of said weakened regions circumscribes a substantially less weakened region.

6. A process for selectively aperturing a web material according to Claim 1 wherein said web material is a nonwoven.

7. An apparatus for selectively aperturing a web material, said apparatus comprising
- a web material supply means
- a web weakening means for selectively weakening a plurality of regions of said web material
- a web removal means for removing at least a part of said web material comprised in said weakened regions.

8. An apparatus for selectively aperturing a web material according to Claim 7 wherein said web weakening means comprises a calander roll and an anvil roll, the surface of said calander roll comprising a plurality of protuberances.

9. An apparatus for selectively aperturing a web material according to Claim 9 wherein at least a portion said protuberances comprise at least one aperture on their distal surface.

10. A absorbent article comprising the selectively apertured web material manufactured according to the process of Claim 1.
